# FASCICULE DE BREVET EUROPEEN

(11) **EP 0 999 805 B1**
(45) Date de publication et mention de la délivrance du brevet: **22.08.2001**
(21) Numéro de dépôt: 98940316.7
(22) Date de dépôt: 22.07.1998
(51) Int. Cl.: A61F 2/00, D04B 21/00

(54) **TISSU PROTHETIQUE TRIDIMENSIONNEL AJOURE**
DREIDIMENSIONALE DURCHLÖCHERTE GEWEBEPROTHESE
THREE-DIMENSIONAL OPEN-WORKED PROSTHETIC FABRIC

(30) Priorité: 01.08.1997 FR 9710103
(43) Date de publication de la demande: 17.05.2000
(73) Titulaire: SOFRADIM PRODUCTION, 01600 Trévoux (FR)
(72) Inventeur: ORY, François, Régis, F-69270 Fontaine Saint Martin (FR); THERIN, Michel, F-69002 Lyon (FR); MENEGHIN, Alfredo, F-69400 Villefranche sur Saône (FR)
(74) Mandataire: Guerre, Dominique
(86) Numéro de dépôt international: FR9801625
(87) Numéro de publication internationale: WO9905990

(56) Documents cités:
- EP-A- 0 621 014
- EP-A- 0 797 962
- US-A- 3 124 136
- US-A- 4 769 038
- US-A- 5 569 273

## Description

La présente invention concerne un tissu tridimensionnel, utile notamment en chirurgie pariétale et/ou viscérale, mais pouvant également trouver application en d'autres domaines de la chirurgie.

La présente invention sera plus particulièrement décrite par rapport à un tissu prothétique du type précité, destiné à être utilisé pour la réparation de hernies ou d'éventrations.

Depuis longtemps, les chirurgiens ont utilisé des tissus prothétiques plats, c'est-à-dire à deux dimensions, pour réparer ou remplacer toute partie du corps, telle qu'une aponévrose, ou une paroi musculaire, détruite ou endommagée, par exemple suite à un traumatisme. Par conséquent, il existe aujourd'hui un grand nombre de tels tissus prothétiques, fabriqués ou obtenus selon divers procédés, par exemple tissage, tricotage, ou moulage, et qui ont été conçus pour remplir souvent des fonctions spécifiques au sein du corps dans lequel ils sont implantés.

De tels tissus bidimensionnels sont par exemple décrits par les documents US-C-5 569 273 et 3 124 136.

Toutefois, de tels tissus présentent plusieurs inconvénients, liés à la structure même du tissu, ou au choix du matériau de base utilisé pour constituer les fils, ou encore à la façon dont le tissu prothétique est fabriqué. Ainsi, les tissus prothétiques connus en matière synthétique polymère par exemple, sont relativement peu souples.

Il est également connu que la réaction tissulaire à un implant est le résultat d'un processus normal de cicatrisation. Tout traumatisme chirurgical tissulaire est suivi d'une cascade d'événements physiologiques dont les principaux temps sont les suivants:
- t0 : traumatisme chirurgical, effraction capillaire ;
- t0 plus quelques minutes : coagulation, réseau fibrineux, libération de facteurs chimiotactiques ;
- t0 plus 12 à 48h : afflux leucocytaire à dominante polynucléaire ;
- t0 plus 4 à 8 jours : afflux fibroblastique ;
- t0 plus 24h à 5 jours : afflux leucocytaire à dominante macrophagique ;
- t0 plus 8 à 21 jours : différenciation conjonctive de la réaction cicatricielle ;
- t0 plus 15 à 180 jours : remodelage cicatriciel au contact de l'implant.

Même si les mécanismes exacts sont pour certains encore inconnus, notamment en ce qui concerne le déterminisme de l'intensité de la réaction, il apparaît donc que les 8 premiers jours sont déterminants puisqu'ils conditionnent l'afflux fibroblastique.

En tissus non osseux, l'équilibre de la réaction conduit à la formation d'une membrane fibroconjonctive qui constitue l'interface entre le matériau implanté et le tissu sain environnant. Quelque soit le type d'implant, la zone sous influence directe d'un matériau conventionnel biocompatible est au minimum d'environ 50 *µ*m.

Par ailleurs, dans le traitement des insuffisances pariétales (hernies et éventrations principalement), le tissu prothétique a pour vocation d'apporter un complément de résistance mécanique à la reconstruction chirurgicale, et est d'autant plus efficace, et sa tolérance locale d'autant meilleure, que son intégration tissulaire est intime et précoce.

La Demanderesse a établi par ses propres études dont les résultats l'ont conduit à la présente invention, que plusieurs paramètres influencent la réponse tissulaire à un implant prothétique, à savoir :
- le matériau constitutif de la prothèse et ses éventuels produits de dégradation en milieu biologique, qui ne doivent pas induire d'effet toxique ou contraire à l'effet recherché. Dans le cas d'un tissu prothétique implanté à long terme, le vieillissement du matériau et ses conséquences (usure, relarguage de composants, etc...) sont le facteur le plus difficile à anticiper ; seules des matières premières validées de longue date apportent un maximum de sécurité ;
- l'organisme ne voyant pour ainsi dire que la surface du matériau, les propriétés de celui-ci revêtent une importance non négligeable. Parmi l'ensemble des paramètres de surface, l'énergie de surface et la rugosité tiennent un rôle important. En effet, lorsque l'on recherche à promouvoir l'adhésion cellulaire, la surface doit être hydrophile (énergie de surface élevée) et lisse à l'échelle d'une cellule (de l'ordre du micron) ;
- seule la porosité accessible par l'organisme est utile en ce qui concerne l'ancrage du tissu prothétique. Cette porosité dans un volume donné doit être interconnectée, et les interstices d'interconnexion doivent être suffisants pour une pénétration cellulaire significative (de l'ordre de 20 à 80 *µ*m environ) , et pour une différenciation tissulaire (100 à 300 *µ*m constituent en général un minimum pour une différenciation complète). Il a été rappelé précédemment que la distance minimum pour mettre à l'écart la réaction tissulaire de l'influence directe du matériau prothétique est de l'ordre de 50 *µ*m, ce qui signifie que pour des tailles de porosité inférieures à 100 *µ*m, le tissu de réhabitation va entièrement être sous l'influence de la présence de l'implant, avec peu de possibilité pour une différenciation tissulaire achevée ;
- la vascularisation et l'environnement biomécanique du site receveur conditionnent l'intensité de la réponse tissulaire. Un site richement vascularisé (peau, muscles, etc...) réagira plus vite et plus intensément que des tissus moins vascularisés (chambre antérieure de l'oeil, os, etc...). Par ailleurs, la nature même du site receveur conditionne la capacité de régénération à l'identique du tissu lésé. L'os, les tissus conjonctifs, les muqueuses, certains parenchymes (le foie par exemple) peuvent se régénérer à l'identique sans cicatrice fibreuse importante. En revanche, d'autres tissus très spécialisés (muscles, tissus nerveux, etc...) ont perdu toute capacité de régénération, la cicatrisation de ces tissus ne se faisant alors que par fibrose ;
- le traumatisme chirurgical constitue l'un des principaux facteurs déclenchants de la réaction en cascade décrite précédemment. Plus celui-ci sera important, plus la réaction sera intense et ses conséquences prononcées (délai de cicatrisation, séquelles fibreuses, douleurs, etc...).

Compte tenu de ce qui précède, un des objectifs d'un bon tissu prothétique est une intégration tissulaire la plus rapide possible, procurant un ancrage mécaniquement satisfaisant sans fibrose extensive, source d'inconfort et de douleur.

Afin d'essayer d'atteindre cet objectif, la Demanderesse a conçu, fabriqué et commercialisé sous la désignation "PAT" ou "TET", un tissu prothétique tridimensionnel, comprenant deux faces opposées, poreuses ou relativement rugueuses, séparées l'une de l'autre par l'épaisseur du tissu, mais reliées l'une à l'autre par des fils de liaison, dont la texture dépend du mode d'obtention du tissu, par exemple par tissage et/ou tricotage.

Un tel tissu prothétique tridimensionnel est par exemple décrit par le document EP-A-0 621 014, comme partie centrale d'un ensemble prothétique comprenant par ailleurs des flancs en tricot simple couche.

Dans certains cas, l'armure du tissu prothétique détermine dans son épaisseur une multiplicité d'alvéoles ou canaux transversaux, sensiblement parallèles les uns aux autres, débouchant de part et d'autre dudit tissu sur les deux faces poreuses respectivement, de telle sorte que l'on peut alors parler de tissu ajouré, permettant un cheminement direct des cellules réparatrices, à partir de chacune des faces du tissu.

Dans la présente demande certains termes ont la signification suivante :
- par "tissu" on entend un assemblage de fils, obtenu notamment par tricotage et/ou tissage ;
- par "face poreuse", on entend une face du tissu qui présente de nombreux petits orifices, ou trous débouchant dans sa surface, lui conférant de ce fait un aspect rugueux.

Ce type de tissu a largement démontré son efficacité, notamment en termes d'intégration la plus rapide possible dans le site anatomique récepteur, en procurant un ancrage mécaniquement satisfaisant sans fibrose extensive ou importante.

La présente invention a toutefois cherché à améliorer l'intégration tissulaire d'un tissu prothétique tridimensionnel tel que défini précédemment, tout en baissant son pouvoir inflammatoire intrinsèque.

Conformément à la présente invention comme définie dans la revendication 1, l'armure du tissu détermine également, pour chaque canal ou alvéole transversal, une paroi interne poreuse d'interconnexion avec les canaux voisins.

Préférentiellement, les interstices de passage entre canaux ont une largeur comprise entre 100 et 300 *µ*m.

La porosité de la paroi des canaux est déterminée en particulier par l'agencement textile des fils de liaison, eux-mêmes réalisés à partir de multifilaments de fibres (par exemple en polyester) présentant à titre d'exemple un diamètre compris entre 10 et 15 *µ* m.

Grâce à l'invention telle que précédemment définie, les parois des canaux fournissent une zone d'ancrage pour la réaction fibreuse sous dépendance de l'implant (environs immédiats de chaque fil), ce qui contribue néanmoins à une intégration tissulaire du tissu prothétique relativement intime et précoce.

En effet, la Demanderesse a découvert qu'avec un tissu prothétique tel que précédemment défini, la structure tridimensionnelle ainsi obtenue améliore la recolonisation cellulaire, tout en améliorant l'intégration du tissu, car le volume interne disponible est augmenté. De surcroît, lorsque la section interne de chaque alvéole ou canal est substantiellement exempte de tout fil de liaison, on diminue d'autant la réaction inflammatoire du tissu prothétique in vivo.

Par ailleurs, pour une quantité de matière au mètre carré équivalente, une structure tridimensionnelle telle que définie précédemment permet d'ouvrir plus largement l'intérieur du tissu sur l'extérieur, ce qui est propice à une différenciation d'un tissu conjonctif histologiquement normal au coeur de l'implant. La porosité multidirectionnelle selon l'invention favorise en outre le drainage du site et limite ainsi les risques associés aux collections de liquides (séromes, hématomes, sepsis).

Grâce à l'invention, on améliore les propriétés mécaniques du tissu, sans augmenter la densité locale de matériau prothétique, puisque ce dernier se trouve distribué en volume, selon trois directions.

Une fois le tissu prothétique implanté, les cellules, présentes au centre du volume créé la structure tridimensionnelle selon l'invention, se trouvent à au moins 750 *µ*m de tout matériel prothétique, si les conditions dimensionnelles définies ci-après sont respectées. Ainsi, les cellules colonisatrices sont loin de toute influence pouvant retarder ou perturber les mécanismes de différenciation, tout en étant à moins de un millimètre du tissu receveur, c'est-à-dire proches des éléments fournissant les éléments indispensables à une réhabitation rapide (cellules souches progénitrices, capillaires sanguins, etc...).

Ces conditions permettent d'obtenir un ancrage mécaniquement satisfaisant tout en préservant une différenciation achevée à coeur, telle que rencontrée dans un tissu conjonctif normal.

Dans un mode d'exécution préféré de l'invention, chaque canal transversal débouche sur au moins une face poreuse selon une ouverture de forme hexagonale ou selon une ouverture de forme en goutte d'eau. Plus préférentiellement, chaque canal transversal débouche sur une face poreuse selon une ouverture de forme hexagonale, et sur l'autre face poreuse selon une ouverture de forme en goutte d'eau.

De manière préférentielle, le diamètre moyen des canaux transversaux est égal ou supérieur à 0,3 mm, de préférence compris entre 0,7 mm et 2 mm, et plus préférentiellement compris entre 1,3 mm et 1,7 mm. De la même manière, les canaux transversaux présentent généralement une longueur comprise entre environ 0,5 mm et 3 mm, et de préférence comprise entre 1,6 mm et 2,1 mm, correspondant à l'épaisseur du tissu.

De préférence, l'épaisseur du tissu est au plus égale à 3 mm, et plus préférentiellement comprise entre 1,7 mm et 2,5 mm.

Les fils de liaison sont préférentiellement constitués chacun par une multiplicité de filaments continus d'un matériau biocompatible, par exemple polyester. Plus préférentiellement encore, le tissu comprend deux nappes de fils de chaîne tricotés, déterminant du côté extérieur les deux faces poreuses, reliées entre elles, et de préférence deux nappes de fils de liaison intermédiaire.

Dans un autre mode d'exécution préféré du tissu prothétique conformément à l'invention, chaque canal transversal débouche sur au moins une face poreuse selon une ouverture de type macropore, dont le diamètre est compris entre 0,7 mm et 2 mm, et plus préférentiellement compris entre 1,3 mm et 1,7 mm.

Un autre objet de la présente invention est une prothèse pariétale et/ou viscérale, obtenue avec un tissu tel que défini précédemment.

Encore un autre objet de la présente invention est l'utilisation d'un tissu selon l'invention tel que défini, pour obtenir un produit prothétique à usage chirurgical, notamment pour fabriquer une prothèse pariétale et/ou viscérale.

Préférentiellement, les interstices de passage entre canaux ont une largeur comprise entre 100 et 300 *µ*m.

La présente invention sera mieux comprise par rapport à la description détaillée de quelques modes d'exécution préférés de l'invention, donnée à titre d'exemple, en association avec les figures en annexe, dans lesquelles :
- la **Figure 1** représente une image d'une face d'un tissu prothétique tridimensionnel ajouré conformément à l'invention, prise par microscopie électronique à balayage ;
- la **Figure 2** représente une image en microscopie électronique à balayage du tissu prothétique de la Figure 1, vu de l'autre face ;
- la **Figure 3** représente une image en microscopie électronique à balayage du tissu prothétique de la Figure 2, à échelle agrandie ;
- la **Figure 4** représente une image en microscopie électronique à balayage du tissu prothétique des Figures 1 à 3, en coupe ou selon sa tranche ;
- la **Figure 5** représente un dessin schématique de l'armure de tricotage pour obtenir un tissu prothétique selon les figures 1 à 4 ;
- la **Figure 6** représente un dessin schématique de l'armure de tricotage d'un tissu prothétique selon un autre mode préféré de l'invention.

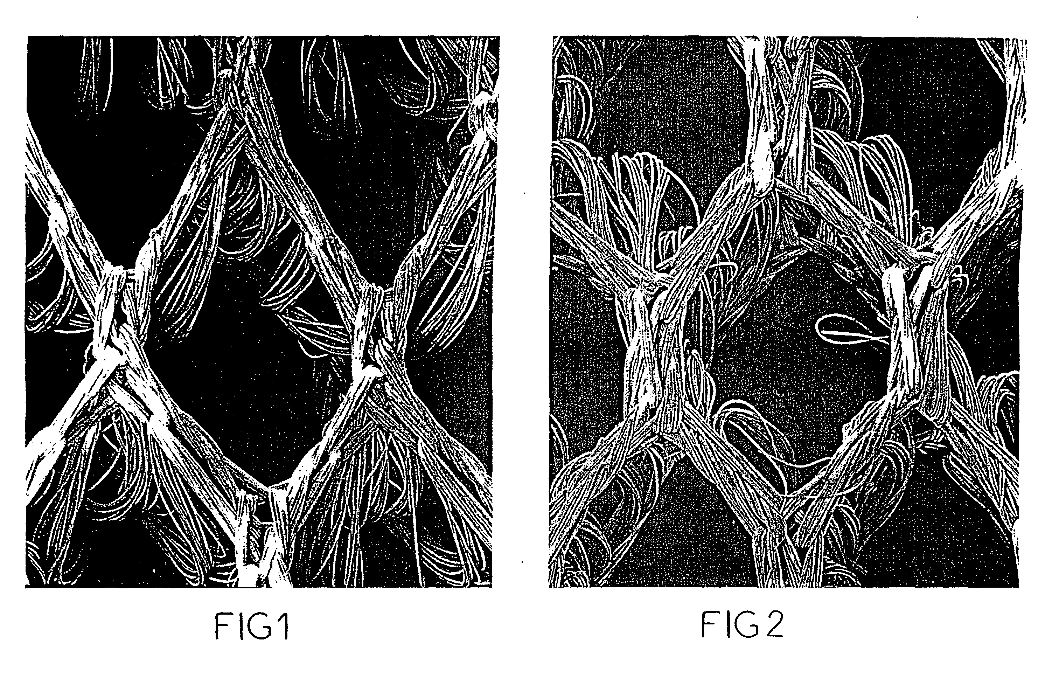

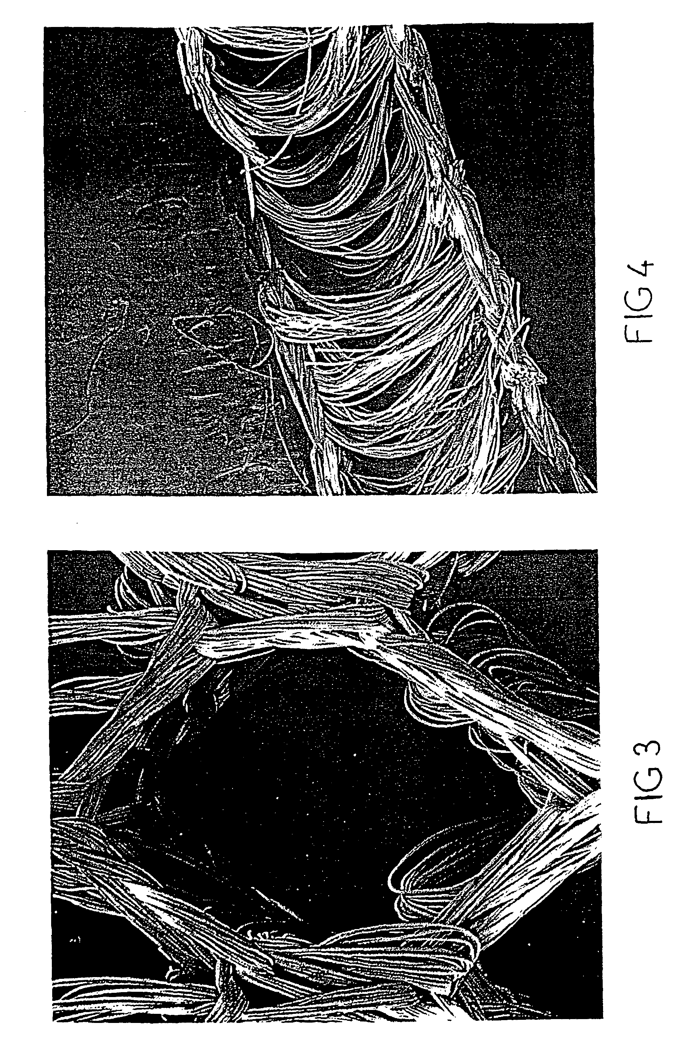

En se référant aux Figures 1 et 2, le tissu prothétique tridimensionnel ajouré selon un premier mode d'exécution préféré de l'invention comprend deux faces indépendantes, l'une présentant des ouvertures en forme de goutte d'eau (cf. Figure 1), et l'autre des ouvertures de forme hexagonale (cf. Figure 2). Les ouvertures d'une même face sont définies par des bords périphériques, formés avec les fils constitutifs du tissu prothétique, qui est en un matériau polymère synthétique ou naturel, résorbable ou non, du type couramment utilisé pour de tels tissus prothétiques, par exemple du polypropylène, polyester, ou polyamide. Dans le mode d'exécution décrit, on utilise de préférence un polyester 50 décitex multifilaments. Comme on peut le voir sur les Figures 1, 2 et 4, les nappes de fils de chaîne tricotés déterminant les faces poreuses sont reliées entre elles par deux nappes de de liaison intermédiaire, qui s'étendent sensiblement perpendiculairement depuis une face vers l'autre face, et qui sont distribués selon les bords périphériques. Les fils de liaison ainsi distribués forment des canaux transversaux, sensiblement parallèles les uns aux autres, dont la section interne est exempte de fils, (cf. Figure 3) ; ces fils relient les ouvertures d'une face aux ouvertures de l'autre face, ce qui confère une structure du type nid d'abeille au tissu prothétique. Ces canaux transversaux sensiblement parallèles les uns aux autres, débouchent de part et d'autre du tissu, sur les deux faces poreuses respectivement.

Conformément à l'invention, les fils de liaison sont disposés en sorte que chaque canal ou alvéole transversal ait une paroi interne poreuse d'interconnexion latérale avec les canaux voisins, ces interstices ayant un diamètre compris entre 100 et 300 *µ*m.

Les canaux transversaux augmentent la vitesse de colonisation cellulaire, une fois le tissu implanté in vivo, car ils facilitent l'acheminement ou l'afflux cellulaire au site de l'implantation. Par ailleurs, la quasi-absence de fils dans le volume même des canaux transversaux permet de baisser la réaction inflammatoire du tissu prothétique, ce qui favorise encore une bonne implantation de ce dernier.

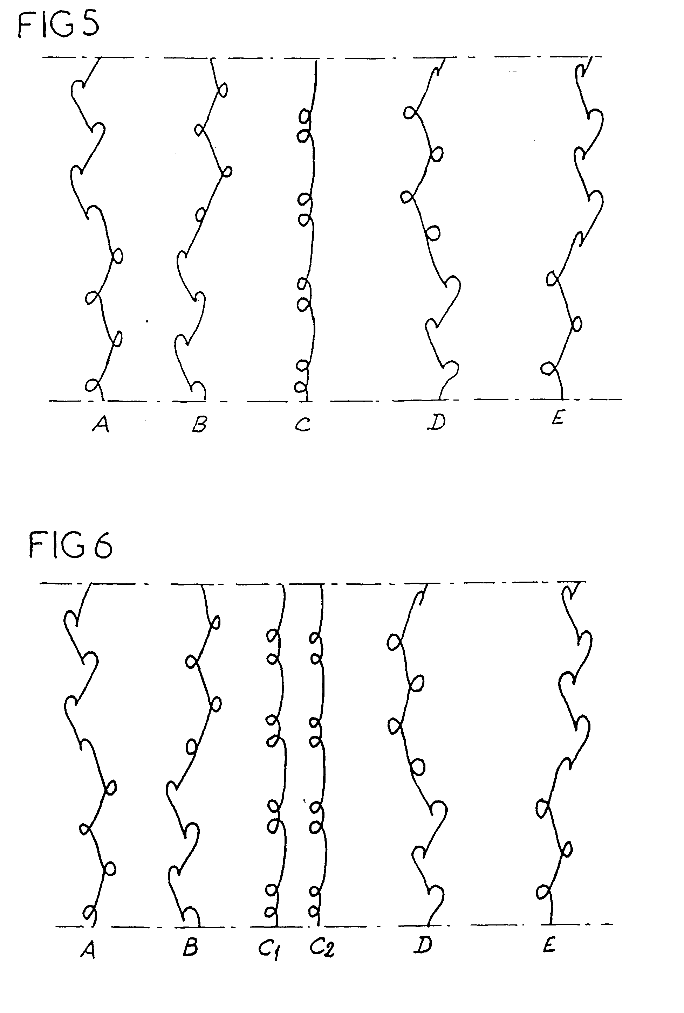

Une telle structure est réalisée par tricotage en chaîne de cinq nappes de fils, et conformément au dessin schématique de la Figure 5. Dans cette figure, chaque nappe de fils est identifiée par une lettre, allant de A à E, le schéma en lui-même utilisant un système de description du tricot à réaliser tout à fait habituel et compréhensible pour l'homme du métier, et qui ne sera pas décrit plus en détails ici. Conformément à la Figure 5, le tissu prothétique préféré selon la présente invention est constitué, comme précédemment décrit, de deux faces poreuses indépendantes. Ces deux faces sont, dans l'exemple donné, elles-mêmes constituées de deux nappes de fils, référencées A,B et D,E respectivement, les nappes A,B donnant une face à ouverture en forme de goutte d'eau, et les nappes D,E donnant une face à ouverture hexagonale. Le tissu prothétique peut être tricoté sur un métier Rachel à double fonture. Dans ce cas, toutes les barres correspondant aux fils A,B et D,E sont enfilées un plein-un vide. La nappe de fils de liaison est représentée par la référence C, et est enfilée pleine. Les différentes nappes A-E de fils sont toutes tricotées en même temps. Ainsi, les fils de liaison sont distribués selon les bords périphériques des ouvertures de chaque face et s'étendent sensiblement perpendiculairement depuis une face vers l'autre face, en ménageant des interstices d'interconnexion latérale avec les autres canaux, et en évitant que des fils de liaison occupent une partie trop importante du volume des canaux transversaux qui sont formés. Le tissu final peut ensuite être stabilisé simplement par passage au four à une température comprise entre environ 170°C et environ 220°C. L'épaisseur du tissu obtenu est de l'ordre de 1,8 mm, et d'un poids d'environ 90 g/m². Ce tissu présente une résistance à la rupture, mesurée selon la norme NFG 07-119, comprise entre environ 18 daN et 30 daN, en chaîne, et comprise entre environ 9 daN et 15 daN, en trame, pour un allongement à la rupture d'environ 25% à 37% en chaîne, et d'environ 60% à 88% en trame.

Dans un autre mode d'exécution préféré de la présente invention, les deux faces du tissu prothétique présentent des ouvertures hexagonales. Un tel tissu peut être obtenu en suivant le schéma de tricotage donné en Figure 6, dans laquelle on retrouve les quatre nappes de fils A,B et D,E, chaque paire constituant une face poreuse indépendante, et reliées entre elles par deux nappes de fils de liaison C1,C2. Dans ce mode d'exécution, toutes les barres, y compris celles des nappes de liaison, sont enfilées un plein-un vide.

## Revendications

1. Tissu tridimensionnel, comprenant deux faces poreuses opposées, séparées l'une de l'autre par l'épaisseur dudit tissu, mais reliées l'une à l'autre par des fils de liaison, comprenant dans son épaisseur une multiplicité d'alvéoles ou canaux transversaux sensiblement parallèles les uns aux autres, débouchant de part et d'autre dudit tissu sur les deux faces poreuses respectivement, caractérisé en ce qu'il présente une structure dans laquelle chaque canal ou alvéole transversal une paroi poreuse d'interconnexion avec les canaux voisins, telle que susceptible d'être obtenue, par tricotage en chaîne, sur un métier RACHEL à double fonture, d'au moins cinq nappes de fils, à savoir quatre nappes A, B, D et E, constituant deux à deux les deux faces respectivement et au moins une nappe (c) de fils de liaison, les barres correspondant aux fils (A,B,D,E) desdites quatre nappes respectivement étant enfilées un plein-un vide.

2. Tissu selon la revendication 1, caractérisé en ce que les fils de liaison sont distribués selon les bords périphériques des ouvertures de chaque face, et s'étendent d'une face à l'autre, en ménageant des interstices d'interconnexion latérale entre canaux.

3. Tissu selon la revendication 1, caractérisé en ce que sa structure comporte deux nappes (C1, C2) de fils de liaison.

4. Tissu selon la revendication 1, caractérisé en ce que le diamètre moyen des canaux transversaux est égal ou supérieur à 0,3 mm, de préférence compris entre 0,7 mm et 2 mm, et plus préférentiellement compris entre 1,3 mm et 1,7 mm.

5. Tissu selon la revendication 1, caractérisé en ce que son épaisseur est au plus égale à 3 mm, et de préférence comprise entre 1,7 mm et 2,5 mm.

6. Tissu selon la revendication 1, caractérisé en ce que la section interne de chaque alvéole ou canal est substantiellement exempte de tout fil de liaison.

7. Tissu selon la revendication 1, caractérisé en ce que les fils de liaison sont constitués chacun par une multiplicité de filaments de fibres d'un matériau biocompatible, par exemple polyester.

8. Tissu selon la revendication 1, caractérisé en ce que chaque canal transversal débouche sur au moins une face poreuse selon une ouverture de type macropore, dont le diamètre est compris entre 0,7 mm et 2 mm, et plus préférentiellement compris entre 1,3 mm et 1,7 mm.

9. Tissu selon la revendication 1, caractérisé en ce que les interstices de passage entre canaux ont une largeur comprise entre 50 et 300 *µ*m.

## Patentansprüche

1. Dreidimensionales Gewebe, mit zwei gegenüberliegenden porösen Seiten, die durch die Dicke des Gewebes voneinander getrennt, jedoch durch Verbindungsfäden miteinander verbunden sind, und in seiner Dicke mit einer Vielzahl von zueinander etwa parallelen quer verlaufenden Alveolen oder Kanälen, die jeweils beidseits des Gewebes auf den beiden porösen Seiten münden, dadurch gekennzeichnet, daß es eine Struktur aufweist, in der jeder quer verlaufende Kanal oder jede Alveole eine poröse Verbindungswand mit den benachbarten Kanälen aufweist, wie sie durch Kettwirken von zumindest fünf Fadenscharen auf einer RACHEL-Doppelfontur-Strickmaschine erhalten werden kann, nämlich vier Scharen A, B, D und E, die jeweils paarweise die beiden Seiten bilden, und zumindest einer Schar (C) von Verbindungsfäden, wobei die Barren, die den Fäden (A, B, D, E) der vier Scharen entsprechen, 'eines voll-eines leer' eingefädelt werden.

2. Gewebe nach Anspruch 1, dadurch gekennzeichnet, daß die Verbindungsfäden gemäß der Umfangsränder der Öffnungen jeder Seite verteilt sind und sich von einer Seite zur anderen erstrecken, indem sie seitlich Verbindungszwischenräume zwischen den Kanälen ausbilden.

3. Gewebe nach Anspruch 1, dadurch gekennzeichnet, daß seine Struktur zwei Scharen (C1, C2) von Verbindungsfäden aufweist.

4. Gewebe nach Anspruch 1, dadurch gekennzeichnet, daß der mittlere Durchmesser der quer verlaufenden Kanäle gleich oder größer als 0,3 mm ist, vorzugsweise im Bereich von 0,7 mm bis 2 mm und noch bevorzugter im Bereich von 1,3 mm bis 1,7 mm liegt.

5. Gewebe nach Anspruch 1, dadurch gekennzeichnet, daß seine Dicke höchstens gleich 3 mm beträgt, und vorzugsweise im Bereich von 1,7 mm bis 2,5 mm liegt.

6. Gewebe nach Anspruch 1, dadurch gekennzeichnet, daß der Innenquerschnitt jeder Alveole oder jedes Kanals im wesentlichen frei von den Verbindungsfäden ist.

7. Gewebe nach Anspruch 1, dadurch gekennzeichnet, daß die Verbindungsfäden jeweils durch eine Vielzahl von Faserfilamenten aus einem biokompatiblen Material, beispielsweise Polyester, gebildet sind.

8. Gewebe nach Anspruch 1, dadurch gekennzeichnet, daß jeder quer verlaufende Kanal auf zumindest einer porösen Seite gemäß einer Öffnung von der Art einer Makropore mündet, deren Durchmesser im Bereich von 0,7 mm bis 2 mm, und bevorzugter im Bereich von 1,3 mm bis 1,7 mm liegt.

9. Gewebe nach Anspruch 1, dadurch gekennzeichnet, daß die Durchgangszwischenräume zwischen den Kanälen eine Breite im Bereich zwischen 50 und 300 *µ*m aufweisen.

## Claims

1. Three-dimensional fabric, comprising two opposite porous surfaces, separated from each other by the thickness of the the said fabric, but linked to each other by binding threads, comprising, within its thickness, a plurality of transverse channels or cells substantially parallel to one another, emerging on either side of the the said fabric on the two porous surfaces respectively, characterized in that it exhibits a structure in which each transverse channel or cell [lacuna] a porous wall interconnecting with the adjacent channels, such as may be obtained by warp knitting on a double bed RACHEL loom, of at least five layers of threads, namely four layers A, B, D and E, constituting in pairs the two surfaces respectively and at least one layer (C) of binding threads, the bars corresponding to the threads (A, B, D, E) of the the said four layers respectively being threaded one fullone empty.

2. Fabric according to Claim 1, characterized in that the binding threads are distributed along the peripheral edges of the openings of each surface, and extend from one surface to the other, forming lateral interconnecting interstices between channels.

3. Fabric according to Claim 1, characterized in that its structure comprises two layers (C1, C2) of binding threads.

4. Fabric according to Claim 1, characterized in that the mean diametre of the transverse channels is equal to or greater than 0.3 mm, preferably between 0.7 mm and 2 mm and, more preferably, between 1.3 mm and 1.7 mm.

5. Fabric according to Claim 1, characterized in that its thickness is at most equal to 3 mm, and preferably between 1.7 mm and 2.5 mm.

6. Fabric according to Claim 1, characterized in that the internal section of each cell or channel is substantially free of any binding thread.

7. Fabric according to Claim 1, characterized in that the binding threads each consist of a plurality of filaments of fibres of a biocompatible material, for example polyester.

8. Fabric according to Claim 1, characterized in that each transverse channel emerges on at least one porous surface through an opening of macropore type, whose diametre is between 0.7 mm and 2 mm, and more preferably between 1.3 mm and 1.7 mm.

9. Fabric according to Claim 1, characterized in that the passage interstices between channels have a width of between 50 and 300 *µ*m.
